# FASCICULE DE BREVET EUROPEEN

(11) **EP 0 779 787 B1**
(45) Date de publication et mention de la délivrance du brevet: **03.05.2000**
(21) Numéro de dépôt: 96916279.1
(22) Date de dépôt: 26.06.1996
(51) Int. Cl.: A46B 15/00, A61C 19/04

(54) **BROSSE A DENTS ELECTRIQUE MUNIE DE MOYENS POUR LOCALISER UNE PLAQUE DENTAIRE**
ELEKTRISCHE ZAHNBÜRSTE MIT VORRICHTUNG ZUR LOKALISIERUNG VON ZAHNBELAG
ELECTRIC TOOTHBRUSH WITH MEANS FOR LOCATING DENTAL PLAQUE

(30) Priorité: 28.06.1995 FR 9507785; 13.12.1995 EP 95402808
(43) Date de publication de la demande: 25.06.1997
(73) Titulaire: Koninklijke Philips Electronics N.V., 5621 BA Eindhoven (NL)
(72) Inventeur: POLAERT, Rémy, F-94440 Villecresnes (FR); HAZAN, Jean-Pierre, F-94370 Sucy-en-Brie (FR); GOURRIER, Serge, F-75012 Paris (FR)
(74) Mandataire: Charpail, François
(86) Numéro de dépôt international: IB9600608
(87) Numéro de publication internationale: WO9701298

(56) Documents cités:
- EP-A- 0 056 877
- EP-A- 0 593 375
- WO-A-92/06671
- DE-A- 4 426 446
- US-A- 4 023 230
- US-A- 4 290 433
- US-A- 5 306 144

## Description

L'invention concerne une brosse à dents électrique munie de soies de nettoyage pour soins personnels, comprenant:
- des moyens pour émettre un rayonnement excitateur en direction d'une denture,
- des moyens pour détecter un rayonnement retour de luminescence émis en réponse au rayonnement excitateur par des zones de la denture perturbées par des défauts dentaires,
- des moyens à fibres optiques pour conduire le rayonnement excitateur jusqu'à proximité de la denture,
- des moyens électriques pour imposer à la brosse à dents au moins une cadence de brossage.

La présence d'une plaque dentaire est la cause première du développement des caries. La plaque se forme lentement et systématiquement à la surface des dents entre les séances habituelles de brossage. Lorsque le brossage est insuffisant ou mal effectué, l'accumulation de la plaque dentaire, spécialement dans la zone interdentaire, favorise le développement des bactéries susceptibles de former des acides qui attaquent l'émail protecteur des dents et développent des caries dentaires. Il est donc particulièrement avantageux de pouvoir surveiller la plaque dentaire et de l'éliminer pour éviter son accumulation.

Dans la pratique courante, c'est généralement le praticien dentiste qui procède à ce genre de soins. Une tierce personne a en effet toute possibilité d'examiner la denture et d'agir là où c'est nécessaire.

Néanmoins, on connaît le document WO 92/06671 concernant une brosse à dents permettant un autodiagnostic de la présence de la plaque dentaire. Tout d'abord, un produit apte à générer une fluorescence induite sous l'action d'un rayonnement excitateur est dispersé sur la denture. Ce produit a la particularité d'être absorbé sélectivement par les caries et la plaque dentaire. Il peut s'agir d'un additif fluorescent mélangé à un dentifrice de sorte qu'au cours d'un nettoyage des dents l'utilisateur peut constater l'existence de la plaque dentaire. Pour cela, la brosse à dents comporte un émetteur de lumière qui émet le rayonnement excitateur qui est guidé par des fibres optiques jusqu'à proximité de la denture. Les zones perturbées de la denture émettent alors le rayonnement induit qui peut être détecté soit visuellement par l'utilisateur en inspectant sa denture, soit par un capteur qui actionne des moyens visuels ou sonores avertissant automatiquement l'utilisateur en cas de détection d'une plaque dentaire. Pour permettre la détection, il faut procéder au préalable au rinçage de la cavité buccale et de la brosse à dents.

Mais un tel dispositif présente plusieurs inconvénients. En particulier, le capteur est disposé à la base des soies de nettoyage, ce qui le place directement en contact avec le dentifrice, l'additif fluorescent et la salive. Il faut donc qu'un tel capteur actif soit homologué pour un tel usage. De plus, après un certain temps d'utilisation, le capteur peut s'opacifier, par exemple par une couche de dépôts, ce qui réduit ses capacités de détection.

Par ailleurs, le capteur possède des dimensions non négligeables, ce qui nécessite de maintenir un évidement assez large dépourvu de soies de nettoyage pour que le capteur puisse recevoir le rayonnement induit, ce qui nuit à l'efficacité du brossage.

Il en résulte que la disposition et les dimensions du capteur de rayonnement font qu'il capte la globalité du rayonnement induit émis par l'ensemble de la denture. Un tel agencement ne permet pas de localiser finement les zones perturbées par la plaque dentaire, ces zones étant généralement situées aux interstices, donc peu accessibles, car ayant des dimensions réduites de l'ordre de 1 mm de largeur environ. Pour effectuer un nettoyage efficace de ces zones, il est donc nécessaire de les localiser avec précision, c'est-à-dire individuellement et non pas globalement.

Il est bien évident que lorsque le dentifrice avec son additif se trouve dispersé dans la cavité buccale, le procédé décrit ne permet pas de procéder à la détection de la plaque dentaire. En effet, l'ensemble de la cavité buccale émet le rayonnement induit. Il est donc nécessaire de rincer la cavité buccale pour ne conserver que l'additif fluorescent fixé aux zones perturbées de la denture.

Pour arriver à un enlèvement correct de la plaque dentaire et s'assurer de l'efficacité du nettoyage, il est donc nécessaire de répéter une suite d'opérations consistant à brosser les dents avec le dentifrice et son additif fluorescent puis à rincer la cavité buccale ainsi que la brosse à dents elle-même. Ceci constitue une suite d'opérations fastidieuses à réaliser quotidiennement.

Le but de l'invention est donc d'éviter à l'utilisateur d'avoir à effectuer cette suite d'opérations fastidieuses et de permettre néanmoins une localisation précise des zones perturbées.

Ce but est atteint avec une brosse à dents pour laquelle les moyens à fibres optiques récupèrent un faisceau étroit du rayonnement retour et le dirigent sur les moyens de détection lesquels comportent des moyens de filtrage pour séparer au moins une composante de signal utile d'une composante de bruit de fond, la composante de signal utile étant synchrone d'une des cadences de brossage, les moyens de détection transformant le signal utile en un signal de localisation révélant les zones perturbées.

Ainsi l'utilisateur peut déterminer avec précision la localisation des zones sur lesquelles il doit faire porter son effort de brossage.

Les moyens à fibres optiques peuvent être formés d'une fibre optique unique ou d'une répartition de fibres optiques.

Préférentiellement, la répartition de fibres optiques peut avoir la forme d'une bande étroite de fibres optiques, la bande ayant une dimension étroite sensiblement colinéaire avec une direction principale de brossage.

Lorsque le produit fluorescent est disséminé dans la cavité buccale, on conçoit aisément que l'ensemble de la cavité buccale va émettre en réponse le signal de fluorescence. Néanmoins, selon l'invention, il est possible de détecter chaque passage des moyens de détection devant la plaque dentaire qui se situe habituellement dans les zones interdentaires. Un signal modulé utile est créé dans le détecteur par le fait qu'il y a, en alternance, des zones ayant une plaque dentaire et des zones où il n'y a pas de plaque dentaire. Ceci entraîne l'apparition d'un signal lumineux utile qui, du fait de l'existence de la cadence de brossage, est transformé en un signal électrique modulé qui présente une cadence identique ou multiple de la cadence de brossage. A l'aide de moyens de filtrage opérant soit par détection synchrone soit par filtrage sélectif centré sur la cadence de brossage, il est possible de s'affranchir du signal de fluorescence de bruit de fond engendré par le produit fluorescent qui se trouve soit uniformément dispersé dans la cavité buccale, soit entraîné par le mouvement de la brosse à dents et d'obtenir un signal de localisation révélant l'existence de la plaque dentaire.

Un premier type de mise en oeuvre, qui ne nécessite pas la dispersion d'additif sur la denture, consiste à mesurer la rémanence de la luminescence induite par le rayonnement excitateur. On a en effet observé que les défauts dentaires possèdent une luminescence dont la rémanence est différente de celle des parties saines de la denture.

Un second type de mise en oeuvre consiste à utiliser un produit fluorescent par exemple de la fluorescéine dispersée dans un milieu approprié ou une pâte dentifrice ou un gel dentifrice contenant le produit fluorescent. Le rayonnement retour est donc un rayonnement de fluorescence. Ce mode de fonctionnement se distingue du mode précédent notamment en ce que le rayonnement retour est situé dans une gamme de longueurs d'ondes sensiblement différente de celle du rayonnement excitateur. Les mesures consistent dans ce cas à mesurer l'intensité du rayonnement retour.

L'utilisateur peut être averti par un signal sonore et/ou par un signal optique qui se déclenche lorsque les moyens de détection révèlent l'existence d'une zone perturbée.

Pour aider l'utilisateur, les moyens de détection peuvent délivrer un signal de commande qui réagit sélectivement sur la commande du moteur électrique pour modifier le nettoyage des zones repérées par exemple en agissant sur la cadence de brossage.

Ces différents aspects de l'invention et d'autres encore seront apparents et élucidés à partir des modes de réalisation décrits ci-après.

L'invention sera mieux comprise à l'aide des figures suivantes données à titre d'exemples non limitatifs qui représentent:

Figure 1: un schéma du principe de fonctionnement de la brosse à dents selon un premier mode de réalisation de l'invention.

Figure 2: un schéma du principe de fonctionnement de la brosse à dents selon un second mode de réalisation de l'invention.

Figure 3: un schéma général des moyens électriques principaux de la brosse à dents.

Figure 4: une vue de dessus d'un exemple de disposition des fibres optiques par rapport aux soies de nettoyage.

Figure 5: un schéma électrique pour séparer le signal utile du signal de bruit de fond.

Figure 6: un schéma simplifié d'une brosse à dents à brossage commandé électriquement.

Figure 7: une courbe indiquant un signal utile détecté,

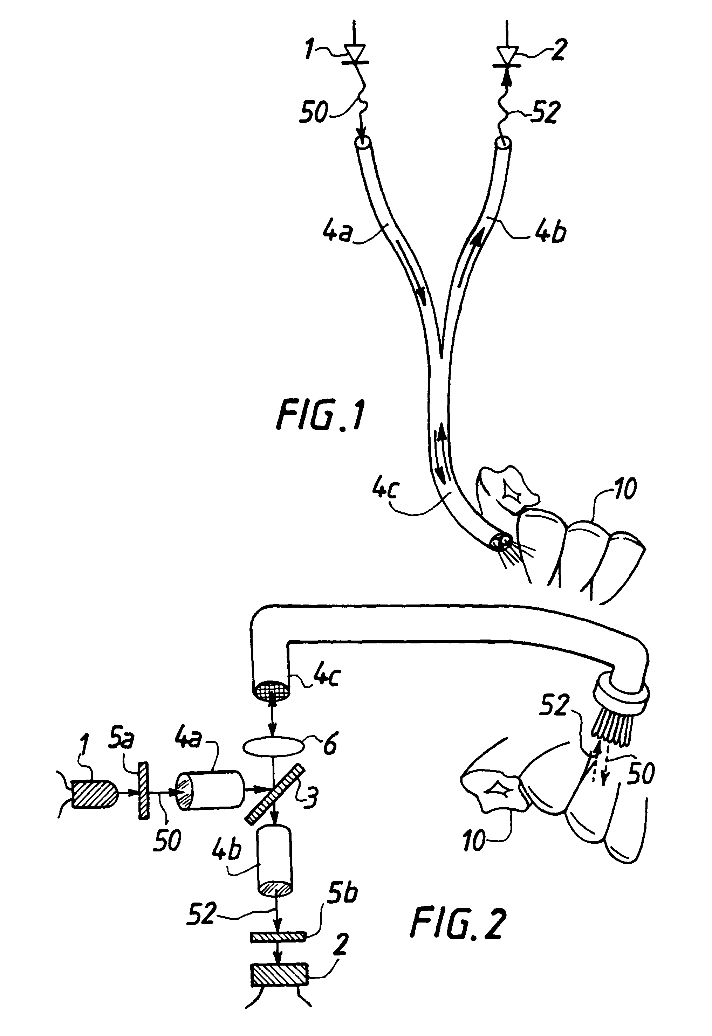

La figure 1 donne le principe de fonctionnement de la brosse à dents selon le premier mode de réalisation de l'invention. Un émetteur de lumière 1 émet un rayonnement excitateur 50 formé d'une lumière bleue ou violette ayant un spectre situé dans la gamme de longueurs d'ondes allant par exemple de 400 à 500 nanomètres environ. Ce rayonnement excitateur est transmis par un conduit à fibres optiques 4a, 4c jusqu'à la denture 10. En réponse au rayonnement excitateur 50 la denture réémet un rayonnement retour 52.

Selon le premier type de mise en oeuvre, le rayonnement retour se situe sensiblement dans la même gamme de longueurs d'ondes que le rayonnement excitateur. Il s'agit dans ce cas d'un signal de luminescence pour lequel on mesure la durée de rémanence de ce rayonnement retour.

Selon le second type de mise en oeuvre, le rayonnement retour se situe dans une gamme de longueurs d'ondes sensiblement différente de celle du rayonnement excitateur. Il s'agit dans ce cas d'un signal de fluorescence pour lequel on mesure principalement son intensité, la fluorescence étant un cas particulier de luminescence. Le produit fluorescent appliqué sur la denture génère le rayonnement retour dans une autre partie du spectre de lumière, par exemple dans le domaine jaune-vert allant de 500 à 600 nanomètres environ.

Pour chacune de ces mises en oeuvre, le rayonnement retour est conduit, sur un trajet retour, par un conduit à fibres optiques 4c, 4b jusqu'à des moyens de détection de lumière comportant, par exemple, un phototransistor 2. Dans la pratique, préférentiellement, le conduit à fibres optiques est en forme de fourche, avec une première partie pour conduire le rayonnement excitateur et une seconde partie pour conduire le rayonnement retour.

Préférentiellement, on utilise une lumière d'excitation modulée en intensité et à la réception on utilise un circuit à détection synchrone, opérant à la cadence de la modulation, pour éliminer les effets des autres sources d'excitation lumineuse.

La figure 2 donne le principe de fonctionnement de la brosse à dents selon le second mode de réalisation de l'invention. Les mêmes éléments sont identifiés avec les mêmes repères que sur la figure 1. L'émetteur de lumière 1 émet le rayonnement excitateur 50 en direction d'un miroir dichroïque 3. Ce rayonnement excitateur peut être filtré par un filtre 5a qui sélectionne la gamme de longueurs d'ondes appropriée. Un guide de lumière 4a permet de faire arriver le rayonnement excitateur sur le miroir dichroïque. Le miroir 3 dévie le rayonnement excitateur vers un guide de lumière 4c qui fait arriver le rayonnement excitateur 50 jusqu'à proximité de la denture 10. Le rayonnement retour 52 est récupéré par le même guide à fibres optiques 4c qui le conduit, selon un chemin inverse, jusqu'au miroir dichroïque 3. Le rayonnement retour étant situé dans une autre gamme de longueurs d'ondes, il traverse alors le miroir dichroïque pour atteindre le phototransistor 2. Un guide de lumière 4b permet de guider le rayonnement retour. Un filtre 5b approprié peut être placé après le miroir dichroïque 3. Ainsi c'est très précisément la zone qui a reçu le rayonnement excitateur qui peut réémettre le rayonnement retour vers le détecteur de lumière. La localisation des défauts dentaires est donc très précise.

Le second mode de réalisation peut également appliquer le premier ou le second type de mise en oeuvre.

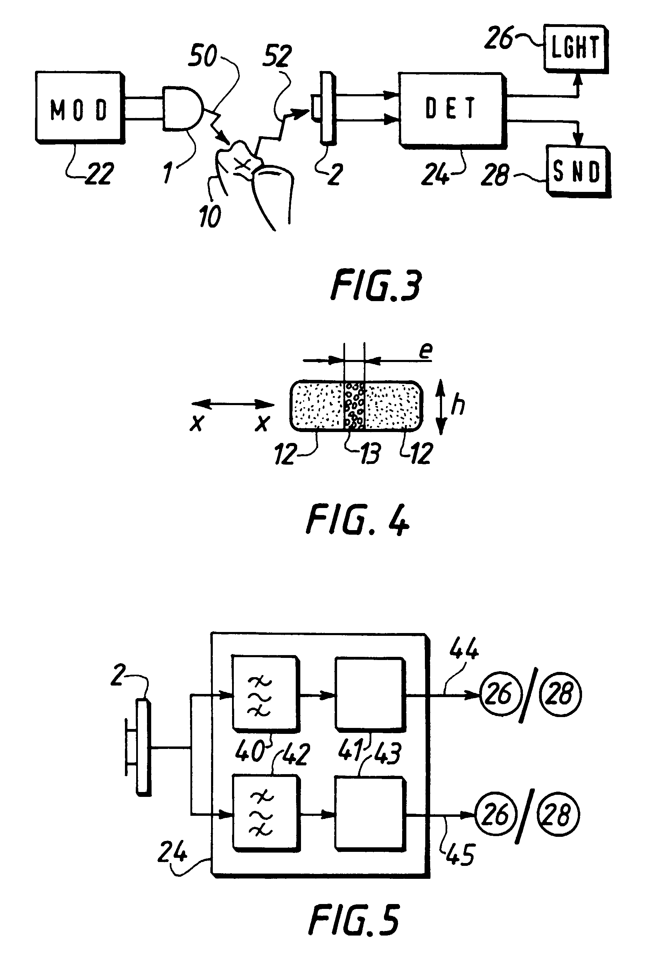

La figure 3 est un schéma général des moyens électro-optiques principaux de la brosse à dents. La partie optique décrite sur les figures 1 et 2 est ici représentée schématiquement par le rayonnement excitateur 50 et le rayonnement retour 52. Des moyens de modulation 22 MOD activent l'émetteur 1 pour émettre le rayonnement excitateur selon une intensité modulée. Le rayonnement excitateur 50 arrive sur la denture 10 qui réémet le rayonnement retour 52 qui est détecté par le photorécepteur 2 qui délivre un signal électrique à des moyens de détection et de démodulation 24 DET. Lorsque le signal électrique reçu est supérieur à un seuil prédéterminé, les moyens 24 actionnent un avertisseur lumineux 26 LGHT ou un avertisseur sonore 28 SND pour alerter l'utilisateur de l'existence d'une plaque dentaire. L'utilisateur peut ainsi accentuer son action de nettoyage.

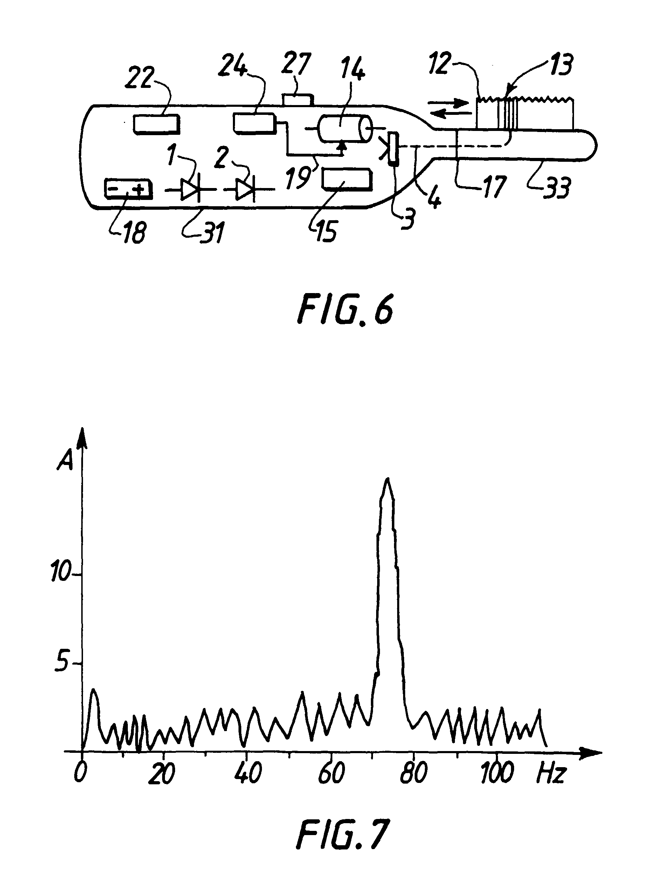

Tous les éléments qui viennent d'être décrits sont réunis dans un boîtier de manière à constituer une brosse à dents manipulable par un utilisateur. La figure 6 représente une telle brosse à dents électrique. Elle comprend un manche 31 renfermant l'émetteur 1, le récepteur 2, le conduit à fibres optiques 4, les moyens de modulation 22, les moyens de démodulation et de détection 24 et le miroir dichroïque 3 pour le second mode de réalisation. En plus, la brosse à dents électrique comporte un moteur électrique 14 activé par un circuit de commande 15. Le manche se prolonge par une partie amincie 33 destinée à être introduite dans la cavité buccale. Cette partie amincie comporte des soies de nettoyage 12 au sein desquelles se trouvent les extrémités 13 des fibres optiques du guide 4. Le moteur électrique 14 donne à la partie amincie 33 un mouvement de va-et-vient permettant d'effectuer le brossage. Le fonctionnement du moteur 14 peut ainsi être contrôlé, c'est-à-dire accéléré, ralenti, arrêté, à l'aide d'un signal de commande 19, en fonction du signal recueilli par le phototransistor relié aux extrémités des fibres optiques placées dans la brosse. La partie amincie 33 peut être désolidarisée du manche 31 selon une jonction 17. L'alimentation électrique est assurée par une pile 18. Un interrupteur marche-arrêt 27 assure la mise en marche et l'arrêt de la brosse à dents.

Les soies de nettoyage 12 et les extrémités 13 des fibres optiques peuvent être disposées comme cela est indiqué sur la figure 4. L'axe XX indique la direction de brossage de la brosse électrique au cours du nettoyage. Préférentiellement, les extrémités 13 sont alors groupées pour former une bande étroite ayant une largeur e et une longueur h, la bande étant disposée de telle manière que la largeur e soit sensiblement parallèle à la direction de la vibration de nettoyage. Ceci présente l'avantage d'examiner chaque dent sur toute sa hauteur dans le sens de la longueur h de la bande et d'examiner l'espace interdentaire avec suffisamment de précision pour bien localiser les défauts dentaires dans le sens de la largeur e de la bande. La bande étroite groupant les extrémités 13 est entourée des soies de nettoyage 12. D'autres dispositions sont également possibles, par exemple ladite bande peut se trouver d'un côté ou de l'autre des soies. Les fibres optiques ont un diamètre (250 micromètres) sensiblement voisin de celui des soies de nettoyage (170 micromètres). Elles peuvent être réalisées, par exemple en polyméthyl méthacrylate. Les fibres et les soies ont sensiblement la même souplesse. En disposant par exemple trois rangées de 20 fibres optiques, on obtient une inspection détaillée même dans les zones interdentaires peu accessibles.

Le faible diamètre des fibres optiques assure une bonne localisation de la plaque dentaire. En effet, la relation entre l'amplitude du signal et la distance séparant les extrémités des fibres optiques de la zone détectée dépend directement du diamètre des fibres optiques utilisées. La sensibilité de détection est une fonction inverse de la distance, la variation de sensibilité selon la distance étant d'autant plus marquée que le diamètre des fibres optiques est plus petit. Ainsi, avec un diamètre de fibres optiques de 250 micromètres, le signal détecté est divisé par 2 pour un éloignement d'environ 250 micromètres. Ces paramètres de proximité et d'étroitesse de bande de fibres optiques contribuent à limiter le faisceau en entrée et sont importants pour permettre aux moyens de détection d'avoir des performances élevées.

Les moyens de détection 24 mesurent soit la rémanence de la luminescence selon le premier type de mise en oeuvre, soit le niveau de fluorescence selon l'autre type de mise en oeuvre.

L'application du produit fluorescent peut être effectué soit en baignant au préalable la cavité buccale avec le produit fluorescent, soit en appliquant une pâte dentifrice ou un gel dentifrice contenant le produit fluorescent. D'autre part, on peut vouloir effectuer la détection des zones perturbées soit après avoir éliminé l'excédent de produit fluorescent disposé dans la cavité buccale, soit durant le brossage en présence du produit fluorescent.

L'invention est principalement concernée par ce dernier cas pour lequel le rayonnement retour comporte une composante continue importante qui perturbe la détection de la plaque dentaire. On peut observer que la composante de signal issue du mélange salive-pâte dentifrice-produit fluorescent, en mouvement dans la cavité buccale, se distingue de la composante de signal issue du produit fluorescent déposé sur la plaque dentaire. En effet, la plaque dentaire étant principalement localisée selon des lignes interstitielles à la jonction entre les dents, il en résulte que le mouvement de va-et-vient imposé aux extrémités des fibres optiques disposées selon une ligne sensiblement perpendiculaire à la direction de ce mouvement, va engendrer une modulation du signal électrique détecté en relation avec la fréquence du brossage. Ce mouvement de brossage comporte par exemple un mouvement rapide de translation à une fréquence voisine par exemple de 75 Hz. Il peut être couplé à un mouvement lent d'oscillation/rotation alternée de ± 3° à raison de 3 oscillations par seconde. Avec ces deux mouvements combinés, en cas de plaque dentaire, le signal détecté présente une première composante autour de 75 Hz et aux fréquences harmoniques supérieures, et une seconde composante autour de 3 Hz. Cette dernière composante correspond plus particulièrement à la partie de la plaque dentaire fixée à la base des dents près des gencives.

En incorporant aux moyens de détection un circuit de filtrage centré sur les fréquences de brossage utilisées, il est possible de détecter la présence de plaque dentaire même si le produit fluorescent, contenu dans la pâte dentifrice, est soit uniformément dispersé dans la cavité buccale, soit entraîné par le mouvement de la brosse à dents.

La figure 5 indique que pour détecter la composante à la fréquence du mouvement de translation, les moyens de détection comportent un premier filtre passe-bande 40 centré sur cette fréquence, suivi d'un étage 41 de détection/redressement qui délivre le signal 44 de localisation relatif à cette fréquence. En outre, les moyens de détection peuvent comporter un second filtre passe-bande 42 centré sur la fréquence du mouvement d'oscillation-rotation, suivi d'un second étage 43 de détection-redressement qui délivre le signal 45 de localisation relatif à cette seconde fréquence.

La figure 7 montre une courbe de l'amplitude A du signal délivré par la sortie du phototransistor 2 en fonction de la fréquence en Hertz. On observe que le signal comporte des pics centrés sur 75 Hz et sur 3 Hz mélangés à du bruit de fond. En isolant par filtrage chacun de ces pics, on construit ainsi le ou les signaux de localisation qui peuvent être utilisés pour actionner les moyens 26, 28 avertissant l'utilisateur.

Evidemment, un tel filtrage conserve tout sont intérêt même lorsque le bruit de fond est faible, par exemple lorsque l'on procède à la détection des zones perturbées après avoir éliminé l'excédent de produit fluorescent réparti dans la cavité buccale.

## Revendications

1. Brosse à dents électrique munie de soies de nettoyage pour soins personnels, comprenant:
- des moyens (1) pour émettre un rayonnement excitateur (50) en direction d'une denture (10),
- des moyens (2,24) pour détecter un rayonnement retour (52) de luminescence émis en réponse au rayonnement excitateur par des zones de la denture perturbées par des défauts dentaires,
- des moyens à fibres optiques (4) pour conduire le rayonnement excitateur (50) jusqu'à proximité de la denture (10),
- des moyens électriques (14,15) pour imposer à la brosse à dents au moins une cadence de brossage,
caractérisée en ce que les moyens à fibres optiques (4) récupèrent un faisceau étroit du rayonnement retour et le dirigent sur les moyens de détection (2,24) lesquels comportent des moyens de filtrage (5b) pour séparer au moins une composante de signal utile d'une composante de bruit de fond, la composante de signal utile étant synchrone d'une des cadences de brossage, les moyens de détection (2,24) transformant le signal utile en un signal de localisation révélant les zones perturbées.

2. Brosse à dents électrique selon la revendication 1, caractérisée en ce que les moyens à fibres optiques (4) sont formés d'une fibre optique unique ou d'une répartition de fibres optiques.

3. Brosse à dents électrique selon la revendication 2, caractérisée en ce que la répartition de fibres optiques (4) forme une bande étroite de fibres optiques, la bande ayant une dimension étroite sensiblement colinéaire avec une direction principale de brossage.

4. Brosse à dents électrique selon la revendication 1 caractérisée en ce que les moyens de détection (2,24) délivrent un signal de commande qui modifie sélectivement la cadence de brossage des zones repérées.

## Claims

1. An electric toothbrush having cleaning bristles for personal care, comprising:
- means (1) for emitting excitation radiation (50) towards teeth (10),
- means (2, 24) for detecting luminescence return radiation (52) emitted in response to excitation radiation from tooth areas affected by dental defects,
- optical fiber means (4) for guiding the excitation radiation (50) to the proximity of the teeth (10),
- electrical means (14, 15) for imposing on the toothbrush at least one brushing rhythm,
characterized in that the optical fiber means (4) pick up a narrow beam of return radiation and guide it to the detection means (2, 24) which comprise filter means (56) for extracting at least one useful signal component from a background noise component, the useful signal component being in synchronism with one of the brushing rhythms, the detection means (2, 24) converting the useful signal into a location signal revealing the affected areas.

2. An electric toothbrush as claimed in Claim 1, characterized in that the optical fiber means (4) are formed by a single optical fiber or by an arrangement of optical fibers.

3. An electric toothbrush as claimed in Claim 2, characterized in that the arrangement of optical fibers (4) forms a narrow band of optical fibers, the band having a narrow dimension which is substantially collinear with a main brushing direction.

4. An electric toothbrush as claimed in Claim 1, characterized in that the detection means (2, 24) supply a control signal which selectively modifies the brushing rhythm of specified areas.

## Patentansprüche

1. Elektrische Zahnbürste, versehen mit Säuberungsborsten zur persönlichen Pflege, die enthält:
- eine Vorrichtung (1) zum Senden einer Stimulationsstrahlung (50) in Richtung der Zähne (10),
- eine Vorrichtung (2, 24) zum Erfassen einer Lumineszenz-Rückstrahlung (52) als Reaktion zur Stimulationsstrahlung von Zahnbereichen, die von Zahnschäden beeinträchtigt sind,
- eine Vorrichtung aus Glasfasern (4) zum Leiten der Stimulationsstrahlung (50) in die Nähe der Zähne,
- eine elektrische Vorrichtung (14 15), um der Zahnbürste mindestens einen Bürsttakt vorzugeben.
dadurch gekennzeichnet, daß die Glasfaservorrichtung (4) ein schmales Lichtbündel der Rückstrahlung zurückerhält und ihn zur Erfassungsvorrichtung (2, 24) leitet, die eine Filtervorrichtung (56) enthält, um mindestens eine Komponente des Nutzsignals von einer Komponente des Grundgeräuschs zu trennen, wobei die Komponente des Nutzsignals synchron zu einer der Bürsttakte ist und die Erfassungsvorrichtung (2, 24) das Nutzsignal in ein Lokalisierungssignal zum Aufzeigen der beeinträchtigten Bereiche wandelt.

2. Elektrische Zahnbürste nach Anspruch 1, dadurch gekennzeichnet, daß die Glasfaservorrichtung (4) aus einer einzigen Glasfaser oder einer Verteilung an Glasfasern besteht.

3. Elektrische Zahnbürste nach Anspruch 2, dadurch gekennzeichnet, daß die Verteilung der Glasfasern (4) vorzugsweise die Form eines schmalen Glasfaserstreifens haben kann, wobei der Streifen eine zur Haupt-Bürstrichtung weitgehend kolineare Abmessung hat.

4. Elektrische Zahnbürste nach Anspruch 1, dadurch gekennzeichnet, daß die Erfassungsvorrichtung (2, 24) ein Steuersignal abgibt, das bei vorgefundenen Bereichen selektiv den Bürsttakt ändert.
